# EUROPEAN PATENT APPLICATION

(11) **EP 1 609 845 A1**
(43) Date of publication of application: **28.12.2005**
(21) Application number: 04722451.4
(22) Date of filing: 22.03.2004
(51) Int. Cl.: C11B 9/00, A61K 7/46, C07D 309/20

(54) **PERFUME COMPOSITIONS AND 3,6-DICYCLOPENTYL-DELTA-VALERO- LACTONE**

(30) Priority: 31.03.2003 JP 2003096287
(71) Applicant: ZEON CORPORATION, Tokyo 100-8246 (JP)
(72) Inventor: WATANABE, K., Zeon Corporation, Chiyoda-ku, Tokyo 100-8323 (JP)
(74) Representative: Albrecht, Thomas
(86) International application number: PCT/JP2004/003893
(87) International publication number: WO 2004/087852

(57) **Abstract**

A perfume composition containing a 3,6-disubstituted δ-valerolactone having fresh, fruity and floral musk-like fragrance, and 3,6- dicyclopentyl-δ-valerolactone. Specifically, the perfume composition contains a 3,6-disubstituted δ-valerolactone represented by formula [1]: 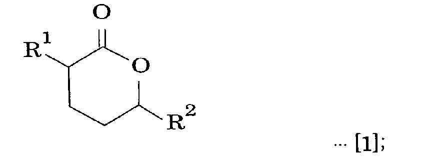 wherein at least one of R¹ and R² represents cyclopentyl group, cyclopentenyl group, cyclohexyl group or cyclohexenyl group and, when only one of R¹ and R² represents cyclopentyl group, cyclopentenyl group, cyclohexyl group or cyclohexenyl group, the other of R¹ and R² represents an alkyl group having 1 to 10 carbon atoms, an alkenyl group having 2 to 10 carbon atoms, an alkynyl group having 2 to 10 carbon atoms, an aryl group having 6 to 10 carbon atoms, an aralkyl group having 6 to 10 carbon atoms or an alkylaryl group having 6 to 10 carbon atoms; and aforesaid 3,6-Dicyclopentyl-δ-valerolactone is represented by formula [2]:

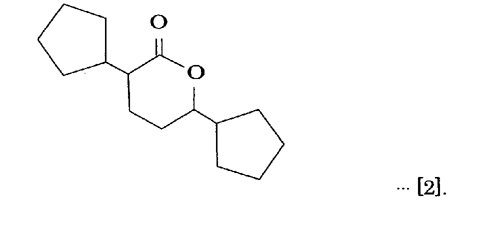

## Description

### TECHNICAL FIELD

The present invention relates to a perfume composition and 3,6-dicyclopentyl-δ-valerolactone. More specifically, the present invention relates to a perfume composition containing a 3,6-disubstituted δ-valerolactone having cyclopentyl group, cyclopentenyl group, cyclohexyl group or cyclohexenyl group at least at one of the 3-position and the 6-position and having fresh, fruity and floral musk-like fragrance, and 3,6-dicyclopentyl-δ-valerolactone.

### BACKGROUND ART

In general, a substance utilized for providing fragrance is called a perfume. The perfume is divided into natural perfumes and synthetic perfumes based on the raw material. The natural perfume is said to include 1,500 types or more of the perfumes, and 100 to 150 types among the natural perfumes are useful in the market. The synthetic perfumes are manufactured by using cheap and abundant natural substances and petroleum-based chemical products as the raw materials. The synthetic perfume is said to include 6,000 types or more of the perfumes, and 500 to 600 types among the synthetic perfumes are frequently used. Since natural perfumes are, in general, more expensive than synthetic perfumes, the effort to develop synthetic perfumes has been continued, and the number of the synthetic perfumes is increasing every year.

The preference of users and consumers to the perfume products described above is varied depending on the age, the sex and the like. The number of product types and the object of the use are rapidly expanding in accordance with this variety. Perfumes with delicate varieties in the basic tone, the depth, the width and the feel of volume in the fragrance are required.

A component of a perfume is one of physiologically active substances. It is known that, when the chemical structure of the component is modified, the fragrance felt by a person differs from the mother compound delicately or, occasionally, to a great degree. Therefore, it is important for obtaining a new perfume that analogues and derivatives of known perfume compounds are synthesized and their fragrance is evaluated.

The present invention has an object of providing a perfume composition containing a 3,6-disubstituted δ-valerolactone having cyclopentyl group, cyclopentenyl group, cyclohexyl group or cyclohexenyl group at least at one of the 3-position and the 6-position and having fresh, fruity and floral musk-like fragrance, and 3,6-dicyclopentyl-δ-valerolactone.

### DISCLOSURE OF THE INVENTION

As the result of intensive studies by the present inventors to achieve the above object, it was found that a 3,6-disubstituted δ-valerolactone having cyclopentyl group, cyclopentenyl group, cyclohexyl group or cyclohexenyl group at least at one of the 3-position and the 6-position has the fresh, fruity and floral musk-like fragrance. The present invention has been completed based on the knowledge.

The present invention provides:
(1) A 3,6-disubstituted δ-valerolactone represented by following formula [1]: 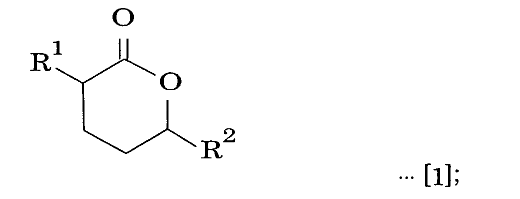 wherein at least one of R¹ and R² represents cyclopentyl group, cyclopentenyl group, cyclohexyl group or cyclohexenyl group and, when only one of R¹ and R² represents cyclopentyl group, cyclopentenyl group, cyclohexyl group or cyclohexenyl group, the other of R¹ and R² represents an alkyl group having 1 to 10 carbon atoms, an alkenyl group having 2 to 10 carbon atoms, an alkynyl group having 2 to 10 carbon atoms, an aryl group having 6 to 10 carbon atoms, an aralkyl group having 6 to 10 carbon atoms or an alkylaryl group having 6 to 10 carbon atoms;
(2) 3,6-Dicyclopentyl-δ-valerolactone expressed by following formula [2]: 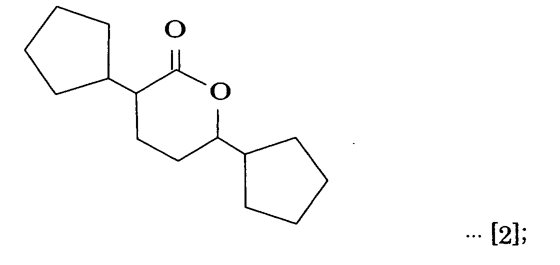
(3) A perfume composition containing a 3,6-disubstituted δ-valerolactone represented by following formula [1]: 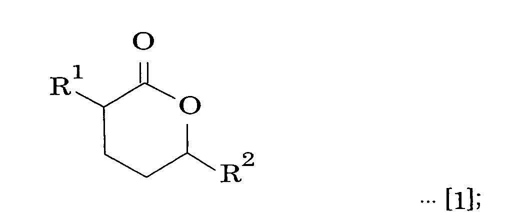 wherein at least one of R¹ and R² represents cyclopentyl group, cyclopentenyl group, cyclohexyl group or cyclohexenyl group and, when only one of R¹ and R² represents cyclopentyl group, cyclopentenyl group, cyclohexyl group or cyclohexenyl group, the other of R¹ and R² represents an alkyl group having 1 to 10 carbon atoms, an alkenyl group having 2 to 10 carbon atoms, an alkynyl group having 2 to 10 carbon atoms, an aryl group having 6 to 10 carbon atoms, an aralkyl group having 6 to 10 carbon atoms or an alkylaryl group having 6 to 10 carbon atoms;
(4) The perfume composition described in (3), wherein at least one of R¹ and R² represents cyclopentyl group and, when only one of R¹ and R² represents cyclopentyl group, the other of R¹ and R² represents an alkyl group having 1 to 10 carbon atoms;
(5) The perfume composition described in (3), wherein at least one of R¹ and R² represents cyclopentyl group and, when only one of R¹ and R² represents cyclopentyl group, the other of R¹ and R² represents n-pentyl group;
(6) The perfume composition described in (3), wherein R¹ and R² represent cyclopentyl groups;
(7) The perfume composition described in (3), which contains the 3,6-disubstituted δ-valerolactone in the concentration from 0.1 to 99 % by weight; and
(8) A process for producing the 3,6-disubstituted δ-valerolactone, as defined in (1), which includes oxidizing a 2,5-disubstituted cyclopentanone represented by following formula [3]: 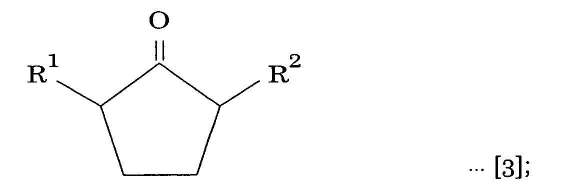
wherein at least one of R¹ and R² represents cyclopentyl group, cyclopentenyl group, cyclohexyl group or cyclohexenyl group and, when only one of R¹ and R² represents cyclopentyl group, cyclopentenyl group, cyclohexyl group or cyclohexenyl group, the other of R¹ and R² represents an alkyl group having 1 to 10 carbon atoms, an alkenyl group having 2 to 10 carbon atoms, an alkynyl group having 2 to 10 carbon atoms, an aryl group having 6 to 10 carbon atoms, an aralkyl group having 6 to 10 carbon atoms or an alkylaryl group having 6 to 10 carbon atoms.

### THE MOST PREFERRED EMBODIMENT TO CARRY OUT THE INVENTION

As the first embodiment, the present invention provides a perfume composition containing a 3,6-disubstituted δ-valerolactone represented by following formula [1]:

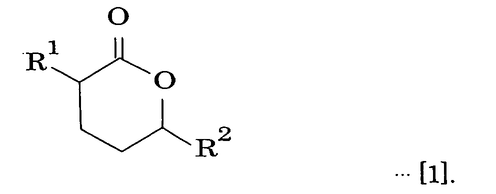

In the above formula [1], at least one of R¹ and R² represents cyclopentyl group, cyclopentenyl group, cyclohexyl group or cyclohexenyl group and, when only one of R¹ and R² represents cyclopentyl group, cyclopentenyl group, cyclohexyl group or cyclohexenyl group, the other of R¹ and R² represents an alkyl group having 1 to 10 carbon atoms, an alkenyl group having 2 to 10 carbon atoms, an alkynyl group having 2 to 10 carbon atoms, an aryl group having 6 to 10 carbon atoms, an aralkyl group having 6 to 10 carbon atoms or an alkylaryl group having 6 to 10 carbon atoms. When R¹ represents cyclopentyl group, it is preferable that R² represents cyclopentyl group, cyclohexyl group or an alkyl group having 4 to 6 carbon atoms, more preferably cyclopentyl group or an alkyl group having 5 carbon atoms, still more preferably cyclopentyl group or n-pentyl group and most preferably cyclopentyl group. When R² represents cyclopentyl group, it is preferable that R¹ represents cyclopentyl group, cyclohexyl group or an alkyl group having 4 to 6 carbon atoms, more preferably cyclopentyl group or an alkyl group having 5 carbon atoms, still more preferably cyclopentyl group or n-pentyl group and most preferably cyclopentyl group.

As the second embodiment, the present invention provides 3,6-dicyclopentyl-δ-valerolactone expressed by following formula [2]:

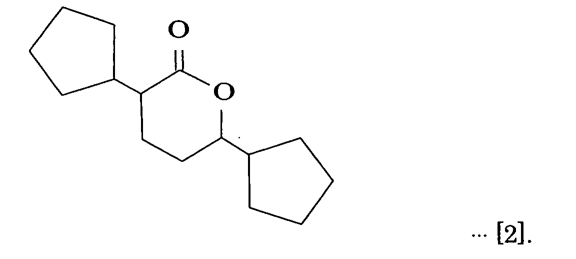

In the present invention, the process for producing the 3,6-disubstituted δ-valerolactone represented by the formula [1] is not particularly limited. For example, the 3,6-disubstituted δ-valerolactone represented by the formula [1] can be obtained via oxidation reaction of a 2,5-disubstituted cyclopentanone represented by formula [3]:

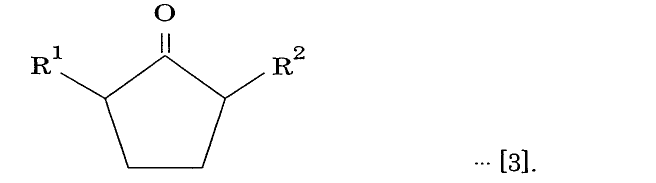

When the 2,5-disubstituted cyclopentanone represented by the formula [3] is, for example, a compound in which both R¹ and R² represent cyclopentyl group, the oxidation of the 2,5-disubstituted cyclopentanone can be conducted, for example, as described in Japanese Patent Application Laid-Open No. 2001-261609 and the United States Patent Application Laid-Open No. 20030012799 in accordance with the reaction route shown in formula [4]:

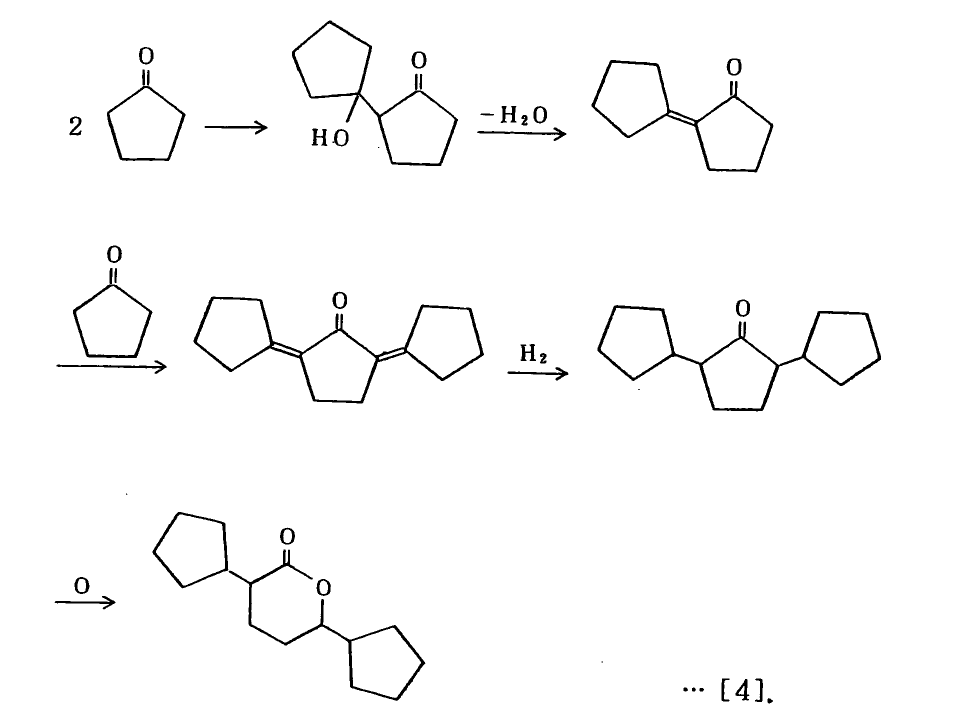

Specifically, 2-cyclopentylidenecyclopentanone is obtained by dehydration of 2-(1-hydroxycyclopentyl)cyclopentanone which is obtained by the aldol condensation of two molecules of cyclopentanone in the presence of an acid catalyst or a base catalyst. The obtained 2-cyclopentylidenecyclo- pentanone is reacted with cyclopentanone in the presence of an alkali such as sodium methylate to obtain 2,5-dicyclopentylidenecyclopentanone, which is further converted into 2,5-dicyclopentylcyclopentanone by hydrogenation of the carbon-carbon double bond in the presence of a palladium catalyst or the like. The obtained 2,5-dicyclopentylcyclo- pentanone is subjected to the Baeyer-Villiger oxidation with an inorganic peroxide such as ammonium peroxodisulfate and potassium peroxo- disulfate or an organic peroxide such as m-chloroperbenzoic acid and benzoyl peroxide, whereby 3,6-dicyclopentyl-δ-valerolactone can be obtained.

It is preferable that the amount of the peroxide used for the Baeyer-Villiger oxidation of 2,5-dicyclopentylcyclopentanone is 0.8 to 2 moles per 1 mole of 2,5-dicyclopentylcyclopentanone. Examples of the solvent for the reaction include water, sulfuric acid, acetic acid, propionic acid, valeric acid and dichloromethane. It is preferable that the amount by weight of the solvent is 0.3 to 30 times and more preferably 0.5 to 20 times as much as the amount by weight of 2,5-dicyclopentylcyclopentanone. It is preferable that the reaction temperature is 10 to 80 °C and more preferably 20 to 40 °C. It is preferable in the Baeyer-Villiger oxidation that the peroxide is added dropwise while the solution of 2,5-dicyclopentylcyclopentanone is stirred to prevent rapid elevation of the reaction temperature.

When the 2,5-disubstituted cyclopentanone represented by the formula [3] is a compound in which one of R¹ and R² represents cyclopentyl group and the other of R¹ and R² represents n-pentyl group, the reaction can be conducted, for example, as described in WO 03/011803, in accordance with the reaction route shown in formula [5]:

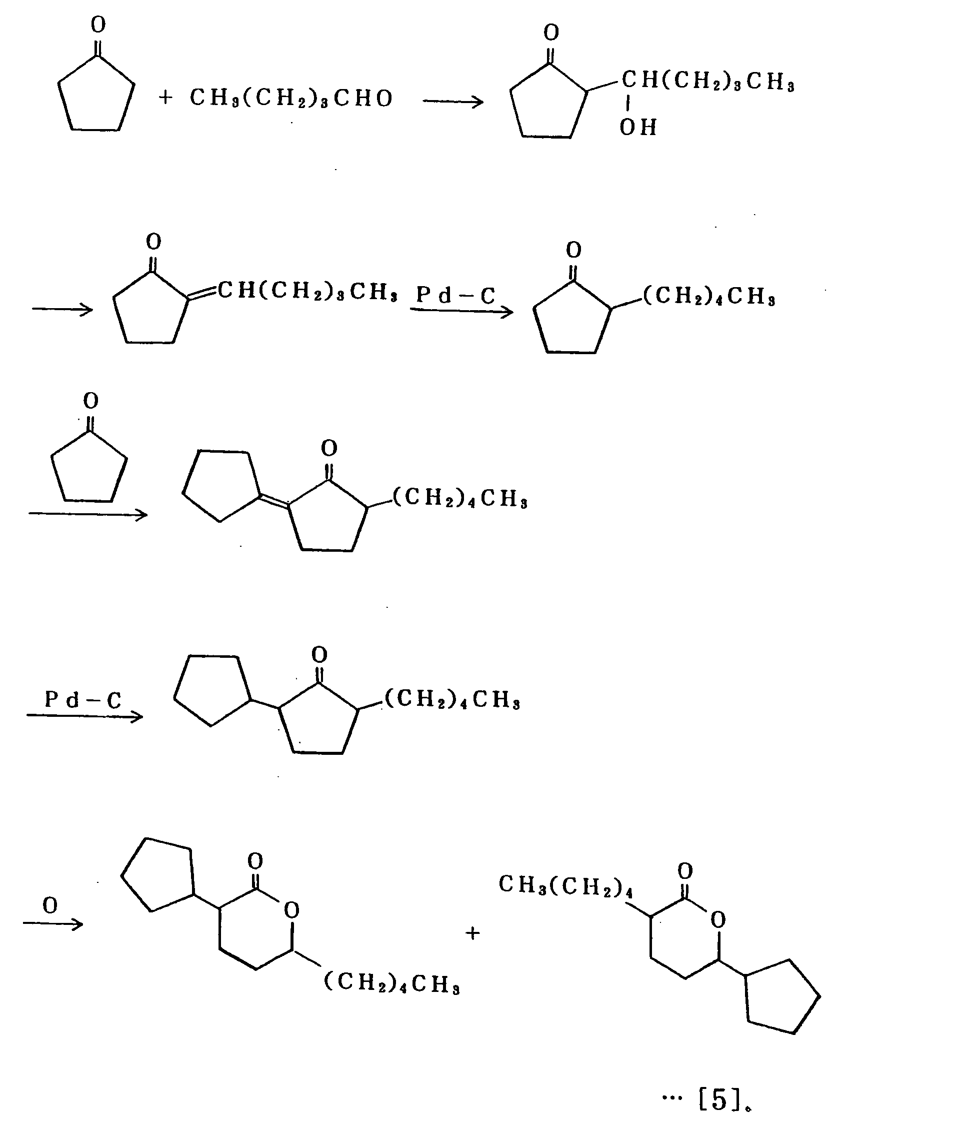

Specifically, 2-pentylidenecyclopentanone is obtained by dehydration of 2-(1-hydroxypentyl)cyclopentanone which is obtained by the aldol condensation of cyclopentanone and valeraldehyde in the presence of an acid catalyst or a base catalyst. The obtained 2-pentylidenecyclo- pentanone is converted into 2-n-pentylcyclopentanone by hydrogenation of the carbon-carbon double bond in the presence of a palladium catalyst or the like. The obtained 2-n-pentylcyclopentanone is reacted with cyclopentanone in the presence of an alkali such as sodium methylate to obtain 2-cyclopentylidene-5-n-pentylcyclopentanone, which is further converted into 2-cyclopentyl-5-n-pentylcyclopentanone by hydrogenation of the carbon-carbon double bond in the presence of a palladium catalyst or the like. The obtained 2-cyclopentyl-5-n-pentylcyclopentanone is subjected to the Baeyer-Villiger oxidation with an inorganic peroxide such as ammonium peroxodisulfate and potassium peroxodisulfate or an organic peroxide such as m-chloroperbenzoic acid and benzoyl peroxide, whereby a mixture of 3-cyclopentyl-6-n-pentyl-δ-valerolactone and 3-n-pentyl-6-cyclopentyl-δ-valerolactone can be obtained.

It is preferable that the amount of the peroxide used in the Baeyer-Villiger oxidation of 2-cyclopentyl-5-n-pentylcyclopentanone is 0.8 to 2 moles per 1 mole of 2-cyclopentyl-5-n-pentylcyclopentanone. Examples of the solvent for the reaction include water, sulfuric acid, acetic acid, propionic acid, valeric acid and dichloromethane. It is preferable that the amount by weight of the solvent is 0.3 to 30 times and more preferably 0.5 to 20 times as much as the amount by weight of 2-cyclopentyl-5-n-pentylcyclopentanone. It is preferable that the reaction temperature is 10 to 80 °C and more preferably 20 to 40 °C. It is preferable in the Baeyer-Villiger oxidation that the peroxide is added dropwise while the solution of 2-cyclopentyl-5-n-pentylcyclopentanone is stirred to prevent rapid elevation of the reaction temperature.

The perfume composition of the present invention contains the 3,6-disubstituted δ-valerolactone represented by the formula [1]. The 3,6-disubstituted δ-valerolactone represented by the formula [1] may be used singly or in combination of two or more types. The composition of the present invention may further contain other perfume components, other solvents and the like, where necessary. It is preferable that the content of the 3,6-disubstituted δ-valerolactone represented by the formula [1] in the composition of the present invention is 0.1 to 99 % by weight and more preferably 0.5 to 90 % by weight.

The other perfume components to be contained in the composition of the present invention are not particularly limited. Examples of the other perfume components include animal-based perfume components such as musk(muscone), civet(civetone), castrium(tetrahydroionone, castrin, acetophenone), ambergris(ambrein, amber oxide and γ-ionone); essential oils such as ambrette seed oil, ylang-ylang oil, oak moss oil, opoponax oil, orris oil, olibanum oil, orange oil, orange flower oil, galbanum oil, clary sage oil, clove oil, grape fruit oil, coriander oil, jasmin oil, spearmint oil, cedarwood oil, geranium oil, thyme oil, tuberose oil, tree moss oil, tonka bean oil, nutmeg oil, neroli oil, neroli bigarade oil, patchouli oil, vanilla oil, hyacinth oil, sandalwood oil, petitgrain oil, bay oil, vetivar oil, bergamot oil, peppermint oil, peru balsam oil, benzoin oil, pepper oil, mandaline oil, mimosa oil, lime oil, lavandin oil, labdanum oil, lavender oil, lemon oil and rose oil; and synthetic perfumes such as ethylene brassylate, eugenol, galaxolid, coumarin, geraniol, vetiveryl acetate, benzyl acetate, linalyl acetate, santalol, citral, citronellol, methyl dihydrojasmonate, aliphatic aldehydes, cis-jasmone, methyl jasmonate, damascone, damascenone, α-terpineol, vanillin, hydroxy- citronellal, phenylacetaldehyde, β-phenylethyl alcohol, α-hexyl- cinnamaldehyde, cis-3-hexenol, heliotropine, vertofix, musk-ketone, methylionone, 1-menthol, linalool, d-limonene, lily aldehyde and rose oxide. These other perfume components may be used singly or in combination of two or more types. In the composition of the present invention, it is preferable that the amount of the other perfume component is 0.1 to 500 parts by weight and more preferably 1 to 200 parts by weight per 1 part by weight of the 3,6-disubstituted δ-valerolactone represented by the formula [1].

The perfume composition of the present invention may be mixed with a solvent component. By mixing with the solvent component, the strength as the perfume can be adjusted, and permeability of the composition into a support can be enhanced when the support is impregnated with the composition. The solvent component is not particularly limited. Examples of the solvent components include ethanol, polyhydric alcohols, paraffin, glycol ethers and esters of phthalic acid. When the support is impregnated with the composition using water as the medium, it is preferable that a surfactant is mixed. The composition of the present invention may contain a fixative as the perfume component to adjust the time of maintaining the fragrance.

The perfume composition of the present invention can be used for detergents, softeners for fiber products, products for softening fiber products, softener products for fibers on dryer use, creams, emulsions, cosmetic powders, talc, body lotions, products for hair dressing, soaps, shampoos, rinses, indoor fragrances, bathing agents, tooth paste, aerosol products and the like.

### EXAMPLES

The present invention will be described more specifically with reference to examples in the following. However, the present invention is not limited to the examples.

For the 3,6-disubstituted δ-valerolactone obtained in Examples, the infrared absorption spectrum, nuclear magnetic resonance spectrum and mass spectrum were measured using a infrared spectrophotometer [manufactured by NIPPON BUNKO KOGYO Co., Ltd; REPORT-100], a nuclear magnetic resonance apparatus [manufactured by VARIAN JAPAN Co., Ltd,; GEMINI 2000] and a mass analyzer [manufactured by NIPPON DENSHI Co., Ltd.; JMS-7000], respectively.

### Example 1 (Synthesis of 3,6-dicyclopentyl-δ-valerolactone)

Into a 500 ml four-necked flask equipped with a stirrer and a reflux condenser, 33 ml of water and 98 ml of a concentrated sulfuric acid were placed under cooling with ice. Then, 66.3 g (0.245 moles) of potassium peroxodisulfate and 110 ml of water were added successively. To the resultant mixture, 33.0 g (0.15 moles) of 2,5-dicyclopentylcyclopentanone obtained in accordance with the process described in Japanese Patent Application Laid-Open No. 2001-261609 was added dropwise over 40 minutes at room temperature, and the obtained mixture was stirred for 40 hours. The reaction mixture was treated by extraction twice each with 150 ml of diethyl ether. The diethyl ether layer was combined, washed successively with a saturated aqueous solution of sodium hydrogencarbonate and a saturated aqueous solution of sodium chloride, and dried with anhydrous magnesium sulfate. The resultant mixture was filtered, and the filtrate was concentrated. The resultant concentrate was treated in accordance with the silica gel column chromatography (the elution solvent: ethyl acetate:n-hexane=1:10 by volume). After recrystallization from a 75 % by weight aqueous solution of ethanol, 13.4 g (56.8 mmoles) of 3,6-dicyclopentyl-δ-valerolactone having a purity of 99 % was obtained as white leaf-shaped crystals. The yield was 38 %.

The obtained 3,6-dicyclopentyl-δ-valerolactone had fresh, fruity and floral musk-like fragrance.
¹H-NMR (500 MHz, CDCl₃, TMS, δ ppm): 1.06-1.33 (m, 4H), 1.33-2.35 (m, 18H), 2.35-2.50 (m, 1H), 3.95-4.13 (m, 1H)
IR (KBr, cm⁻¹): 1720 (C=O)
MS (EI, 70 mV): 236, 193, 167, 150, 121, 108, 96, 81, 67, 54, 41

### Example 2 (Synthesis of 3,6-dicyclopentyl-δ-valerolactone)

Into a 100 ml four-necked flask equipped with a stirrer and a reflux condenser, 50 ml of dichloromethane and 4.4 g (20 mmoles) of 2,5-dicyclopentylcyclopentanone were placed. While the resultant solution was kept at the room temperature, 7.3 g (33.8 mmoles) of a 80 % by weight m-chloroperbenzoic acid was added, and the obtained mixture was stirred at the room temperature for 48 hours. The formed precipitates were removed by filtration, and the organic layer was washed successively with a saturated aqueous solution of sodium hydrogencarbonate and a saturated aqueous solution of sodium chloride, and then dried with anhydrous magnesium sulfate. The resultant mixture was filtered, and the filtrate was concentrated. The resultant concentrate was treated in accordance with the silica gel column chromatography (the elution solvent: ethyl acetate:n-hexane=1:10 by volume). After recrystallization from a 75 % by weight aqueous solution of ethanol, 2.4 g (10.2 mmoles) of 3,6-dicyclopentyl-δ-valerolactone having a purity of 99 % was obtained as white leaf-shaped crystals. The yield was 51 %.

### Example 3 (Synthesis of 3-cyclopentyl-6-n-pentyl-δ-valerolactone and 3-n-pentyl-6-cyclopentyl-δ-valerolactone)

The reaction was conducted in accordance with the same procedures as those conducted in Example 1 except that 33.0 g (0.149 moles) of 2-cyclopentyl-5-n-pentylcyclopentanone obtained in accordance with the process described in WO 03/011803 was used in place of 33.0 g of 2,5-dicyclopentylcyclopentanone. A mixture of 3-cyclopentyl-6-n-pentyl-δ-valerolactone and 3-n-pentyl-6-cyclopentyl-δ-valerolactone, having a purity of 99 %, in an amount of 10.2 g (42.9 mmoles) was obtained as white leaf-shaped crystals. The yield was 28 %.

The obtained mixture of 3-cyclopentyl-6-n-pentyl-δ-valerolactone and 3-n-pentyl-6-cyclopentyl-δ-valerolactone had fresh, sweet and floral musk-like fragrance.

¹H-NMR (500 MHz, CDCl₃, TMS, δ ppm): 0.85 (t, 3H), 1.16-1.44 (m, 8H), 1.44-1.74 (m, 8H), 1.80-2.16 (m, 5H), 2.28-2.48 (m, 1H), 4.00-4.26 (m, 1H)
IR (KBr, cm⁻¹): 1722, 1725 (C=O)
MS (EI, 70 mV): 238, 195, 169, 150, 123, 108, 96, 81, 67, 54, 41

### Example 4 (Synthesis of 3-cyclopentyl-6-n-pentyl-δ-valerolactone and 3-n-pentyl-6-cyclopentyl-δ-valerolactone)

The reaction was conducted in accordance with the same procedures as those conducted in Example 2 except that 4.4 g (19.8 mmoles) of 2-cyclopentyl-5-n-pentylcyclopentanone was used in place of 4.4 g of 2,5-dicyclopentylcyclopentanone. A mixture of 3-cyclopentyl-6-n-pentyl-δ-valerolactone and 3-n-pentyl-6-cyclopentyl-δ-valerolactone having a purity of 99 %, in an amount of 2.0 g (8.4 mmoles) was obtained as white leaf-shaped crystals. The yield was 42 %.

### Example 5 (Preparation of a sweet and floral perfume composition of the semioriental type)

A perfume composition was prepared in accordance with the formulation of perfume components shown in Table 1 and evaluated by seven members of the evaluation panel.

### Comparative Example 1

A perfume composition was prepared in accordance with the same formulation as that shown in Table 1 except that ethylene brasilate was used in place of 3,6-dicyclopentyl-δ-valerolactone and evaluated by seven members of the evaluation panel simultaneously with the evaluation of the perfume composition prepared in Example 5.

The formulation for the perfume composition prepared in Example 5 is shown in Table 1, and the results of the evaluation in Example 5 and Comparative Example 1 are shown in Table 2.

**Table 1**

| Component of perfume | Amount (part by weight) |
|---|---|
| ZEPPIN (NIPPON ZEON) | 2 |
| cis-3-Hexenyl acetate (NIPPON ZEON) | 3 |
| γ-Undecalactone | 4 |
| Ethyl linalool (GIVAUDAN) | 4 |
| Linalyl acetate | 10 |
| Styralyl acetate | 10 |
| AQUANAL (QUEST) | 10 |
| Dihydromyrcenol | 14 |
| Anisaldehyde | 4 |
| Benzyl acetate | 6 |
| Phenylhexanol | 10 |
| Cinnamic alcohol | 10 |
| Dimethylbenzylcarbinyl acetate | 10 |
| Rhodinol | 20 |
| Florosa (QUEST) | 30 |
| Kovanol (TAKASAGO) | 70 |
| SUPER CEPIONATE (NIPPON ZEON) | 90 |
| Phenoxyethyl alcohol | 165 |
| Eugenol | 2 |
| α-Ionone | 10 |
| EBANOL (GIVAUDAN) | 14 |
| 3,6-Dicyclopentyl-δ-valerolactone | 19 |
| ISO·E·Super | 180 |
| Methylionone | 200 |
| AMBROXAN (HENKEL) | 4 |
| Ethylvanillin | 4 |
| Heliotropine | 15 |
| Cyclopentadecanolide | 80 |
| Total | 1,000 |

the perfume composition prepared in Example 1.

The formulation for the perfume composition prepared in Example 5 is shown in Table 1, and the results of the evaluation in Example 5 and Comparative Example 1 are shown in Table 2.

**Table 1**

| Component of perfume | Amount (part by weight) |
|---|---|
| ZEPPIN (NIPPON ZEON) | 2 |
| cis-3-Hexenyl acetate (NIPPON ZEON) | 3 |
| γ-Undecalactone | 4 |
| Ethyl linalool (GIVAUDAN) | 4 |
| Linalyl acetate | 10 |
| Styralyl acetate | 10 |
| AQUANAL (QUEST) | 10 |
| Dihydromyrcenol | 14 |
| Anisaldehyde | 4 |
| Benzyl acetate | 6 |
| Phenylhexanol | 10 |
| Cinnamic alcohol | 10 |
| Dimethylbenzylcarbinyl acetate | 10 |
| Rhodinol | 20 |
| Florosa (QUEST) | 30 |
| Kovanol (TAKASAGO) | 70 |
| SUPER CEPIONATE (NIPPON ZEON) | 90 |
| Phenoxyethyl alcohol | 165 |
| Eugenol | 2 |
| α-Ionone | 10 |
| EBANOL (GIVAUDAN) | 14 |
| 3,6-Dicyclopentyl-δ-valerolactone | 19 |
| ISO·E·Super | 180 |
| Methylionone | 200 |
| AMBROXAN (HENKEL) | 4 |
| Ethylvanillin | 4 |
| Heliotropine | 15 |
| Cyclopentadecanolide | 80 |
| Total | 1,000 |

**Table 2**

| Member of evaluation panel | Example 5 | Comparative Example 1 |
|---|---|---|
| A | elegant top note with feel of volume | weak as overall impression |
| B | powdery with slight sweetness | insufficient in sweetness |
| C | elegantly floral feeling, significantly | sour feeling, significantly |
| D | excellent in quickness | deficient in feel of volume |
| E | more excellent in Example | strong chemical smell |
| F | harmony with combination of fragrances | no depth felt |
| G | elegant, atmosphere of a high grade | stiffness felt |

As shown by the results in Table 2, all members of the evaluation panel evaluated the perfume composition in Example 5 using 3,6-dicyclopentyl-δ-valerolactone as more excellent than the perfume composition in Comparative Example 1 using ethylene brasilate which is produced in a great amount as the macrocyclic musk.

### INDUSTRIAL APPLICABILITY

The perfume composition of the present invention contains the 3,6-disubstituted δ-valerolactone having cyclopentyl group, cyclopentenyl group, cyclohexyl group or cyclohexenyl group at least at one of the 3-position and the 6-position, emits fresh, sweet and floral musk-like fragrance and is useful as the synthetic perfume. The 3,6-dicyclopentyl-δ-valerolactone of the present invention is the compound having fresh, sweet and floral musk-like fragrance.

## Claims

1. A 3,6-disubstituted δ-valerolactone represented by following formula [1]: 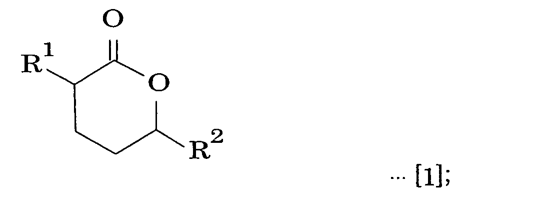 wherein at least one of R¹ and R² represents cyclopentyl group, cyclopentenyl group, cyclohexyl group or cyclohexenyl group and, when only one of R¹ and R² represents cyclopentyl group, cyclopentenyl group, cyclohexyl group or cyclohexenyl group, the other of R¹ and R² represents an alkyl group having 1 to 10 carbon atoms, an alkenyl group having 2 to 10 carbon atoms, an alkynyl group having 2 to 10 carbon atoms, an aryl group having 6 to 10 carbon atoms, an aralkyl group having 6 to 10 carbon atoms or an alkylaryl group having 6 to 10 carbon atoms.

2. 3,6-Dicyclopentyl-δ-valerolactone expressed by following formula [2]:

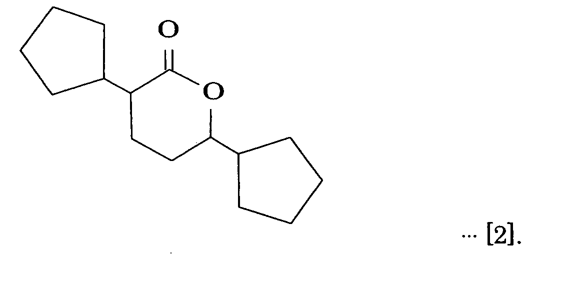

3. A perfume composition comprising a 3,6-disubstituted δ-valerolactone represented by following formula [1]: 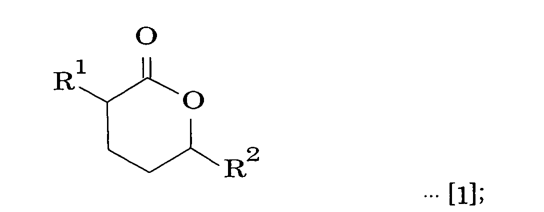 wherein at least one of R¹ and R² represents cyclopentyl group, cyclopentenyl group, cyclohexyl group or cyclohexenyl group and, when only one of R¹ and R² represents cyclopentyl group, cyclopentenyl group, cyclohexyl group or cyclohexenyl group, the other of R¹ and R² represents an alkyl group having 1 to 10 carbon atoms, an alkenyl group having 2 to 10 carbon atoms, an alkynyl group having 2 to 10 carbon atoms, an aryl group having 6 to 10 carbon atoms, an aralkyl group having 6 to 10 carbon atoms or an alkylaryl group having 6 to 10 carbon atoms.

4. The perfume composition according to Claim 3, wherein said at least one of R¹ and R² represents cyclopentyl group and, when said only one of R¹ and R² represents cyclopentyl group, the other of R¹ and R² represents an alkyl group having 1 to 10 carbon atoms.

5. The perfume composition according to Claim 3, wherein said at least one of R¹ and R² represents cyclopentyl group and, when said only one of R¹ and R² represents cyclopentyl group, the other of R¹ and R² represents n-pentyl group.

6. The perfume composition according to Claim 3, wherein said R¹ and said R² represent cyclopentyl groups.

7. The perfume composition according to Claim 3, which comprises the 3,6-disubstituted δ-valerolactone in the concentration from 0.1 to 99 % by weight.

8. A process for producing the 3,6-disubstituted δ-valerolactone, as defined in Claim 1, which comprises oxidizing a 2,5-disubstituted cyclopentanone represented by following formula [3]: 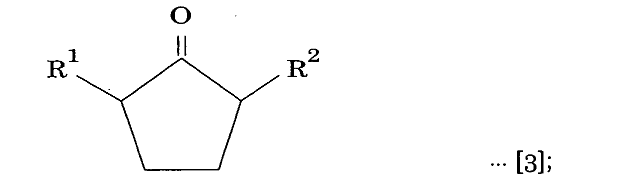 wherein at least one of R¹ and R² represents cyclopentyl group, cyclopentenyl group, cyclohexyl group or cyclohexenyl group and, when only one of R¹ and R² represents cyclopentyl group, cyclopentenyl group, cyclohexyl group or cyclohexenyl group, the other of R¹ and R² represents an alkyl group having 1 to 10 carbon atoms, an alkenyl group having 2 to 10 carbon atoms, an alkynyl group having 2 to 10 carbon atoms, an aryl group having 6 to 10 carbon atoms, an aralkyl group having 6 to 10 carbon atoms or an alkylaryl group having 6 to 10 carbon atoms.
